# EUROPÄISCHE PATENTSCHRIFT

(11) **EP 2 799 002 B1**
(45) Veröffentlichungstag und Bekanntmachung des Hinweises auf die Patenterteilung: **29.04.2020**
(21) Anmeldenummer: 14160414.0
(22) Anmeldetag: 18.03.2014
(51) Int. Cl.: A61B 3/10

(54) **Verfahren und Analysesystem zur Augenuntersuchung**
Method and analysis system for performing ophthalmic examinations
Procédé et système d'analyse pour l'examen des yeux

(30) Priorität: 30.04.2013 DE 102013207987
(43) Veröffentlichungstag der Anmeldung: 05.11.2014
(73) Patentinhaber: Oculus Optikgeräte GmbH, 35582 Wetzlar (DE)
(72) Erfinder: Steinmüller, Andreas, 35435 Wettenberg (DE)
(74) Vertreter: advotec.

(56) Entgegenhaltungen:
- EP-A1- 2 281 500
- WO-A1-2014/028058
- DE-A1-102010 046 500
- US-A1- 2010 014 051
- US-A1- 2010 271 594

## Beschreibung

Die Erfindung betrifft ein Verfahren zur Untersuchung eines Auges mit den Merkmalen des Oberbegriffs des Anspruchs 1 und ein ophthalmologisches Analysesystem mit den Merkmalen des Oberbegriffs des Anspruchs 12.

Verfahren und Vorrichtungen zur Gewinnung von Schnittbildern eines Auges mittels einer Scheimpflugkamera sind aus dem Stand der Technik hinreichend bekannt. So ist beispielsweise aus der DE 10 2005 026 371 eine sogenannte Scheimpflugaufnahmeeinrichtung bekannt, bei der mittels einer Projektionseinrichtung ein Auge mit einem Lichtspalt beleuchtet wird, wobei mittels einer Beobachtungseinrichtung eine Bildaufnahme eines so erzeugten Schnittbildes erfolgt. Die Beobachtungseinrichtung ist im Wesentlichen aus einer Kamera gebildet, bei der sich eine Objektiv- und Bildebene mit einer Objektebene des Schnittbildes in einem gemeinsamen Punkt schneidet. Weiter ist es bekannt, einen so aufgenommenen Bilddatensatz zu speichern und mittels digitaler Bilddatenanalyse, beispielsweise zur Ermittlung optischer Grenzflächen, weiter zu verarbeiten.

Optische Kohärenzinterferometer zur optischen Kohärenztomografie (OCT) sind ebenfalls aus dem Stand der Technik hinreichend bekannt. Im Bereich der Ophthalmoskopie werden diese Analysegeräte regelmäßig für eine detaillierte Untersuchung eines Auges im Bereich eines vorderen Augenabschnitts, eines hinteren, retinalen Augenabschnitts oder auch für einen sogenannten Ganzaugenscan eingesetzt. Bei der optischen Kohärenzinterferometrie wird mit Hilfe eines Interferometers kohärentes Licht zur Bildgebung und Entfernungsmessung an reflektivem und streuendem Augengewebe eingesetzt. Aufgrund von an optischen Grenzflächen des Auges auftretenden Änderungen eines Brechungsindexes und aufgrund von Volumenstreuungen können mittels eines optischen Kohärenzinterferometers messbare Signale gewonnen werden.

Das Grundprinzip der optischen Kohärenzinterferometrie basiert auf der Weißlicht-Interferometrie und vergleicht die Laufzeit eines Signals mit Hilfe eines Interferometers, wie z. B. eines Michelsen-Interferometers. Dabei wird ein optischer Referenzarm mit bekannter optischer Weglänge als Referenz zu einem optischen Messarm bzw. Messstrahl, mit dem ein zu untersuchendes Auge abgetastet wird, herangezogen. Die Interferenz der Signale aus beiden Armen ergibt ein Muster, aus dem eine relative optische Weglänge innerhalb eines Tiefenprofils, welches auch als A-Scan (amplitude-mode scan) bezeichnet wird, herauslesbar ist. In mehrdimensionalen Rasterverfahren kann ein Messstrahl transversal in einer oder zwei Richtungen geführt werden, womit sich ein flächiges Tomogramm, welches auch als B-Scan (brightness-mode scan) bezeichnet wird, ergibt. Auch lässt sich mittels einer Tiefenverstellung eines Messbereichs ein dreidimensionlaes Volumen als sogenannter C-Scan (c-mode scan) aufnehmen.

Anders als bei der konventionellen Lichtmikroskopie ist bei der optischen Kohärenztonometrie die transversale von der longitudinalen Auflösung entkoppelt. Die transversale Auflösung wird durch die nummerische Apertur der verwendeten Optik bestimmt. Die longitudinale räumliche Auflösung in eine Tiefe des Materials hängt dagegen von einer spektralen Breite des verwendeten Lichts ab.

Bei den in der Ophthalmologie verwendeten optischen Kohärenztonometrieverfahren können im Wesentlichen zwei Grundtypen unterschieden werden. Bei einem ersten Typ kann ein Referenzarm eines Interferometers in der Länge verändert und kontinuierlich eine Intensität der Interferenz gemessen werden, ohne dass dabei ein Spektrum berücksichtigt wird. Dieses Verfahren wird nach einer Signalmessung im Zeitbereich als "time domain"-Verfahren bezeichnet. Bei dem zweiten Typ wird zur Bestimmung der Messwerte das Spektrum berücksichtigt und eine Interferenz der einzelnen spektralen Komponenten erfasst. Dieses Verfahren wird als "frequency domain"-Verfahren bezeichnet. Beim "frequency domain"-Verfahren wird kein beweglicher Referenzarm benötigt, wodurch eine einfache und schnelle simultane Messung möglich wird. Insbesondere kann eine vollständige Information über eine Tiefe ermittelt werden. Beim "frequency domain OCT" unterscheidet man wiederum zwei Untergruppen, bei denen einerseits das Signal zeitlich kodiert, also sequenziell aufgenommen wird, oder räumlich kodiert, also räumlich aufgespalten, aber simultan aufgezeichnet wird. Da die mittels der räumlichen Aufspaltung des Signals gewonnene spektrale Information mittels eines Spektrometers erfasst werden kann, wird dieses Verfahren auch "spectral domain OCT" genannt.

Weiter ist ein Verfahren zur Gewinnung eines OCT-Ganzaugenscans bekannt, bei dem ein optisches Kohärenzinterferometer mit einem weiteren bildgebenden Analysesystem kombiniert werden kann. Da mit dem optischen Kohärenzinterferometer tomografische Abbildungen verschiedener Bereiche des Auges bei verschiedenen Referenzarmlängen gescannt werden, müssen diese Teilscans zu einem Gesamtbild des Auges zusammengesetzt werden. Dabei überlappen die Teilscans, um eine passgenaue Kombination von gewonnenen Bilddatensätzen zu ermöglichen. Das weitere Analysesystem kann zur Ergänzung des Ganzaugenscans mit weiteren Bilddaten oder beispielsweise auch topografischen Daten der Hornhaut genutzt werden. Dabei stellt der Ganzaugenscan einen Referenzbilddatensatz dar, der je nach Bedarf einer erforderlichen Augenuntersuchung partiell im Bereich des zu untersuchenden Augenabschnittes mit den Bilddaten des weiteren Analysesystems ergänzt wird. Dies ist insofern vorteilhaft, da mit dem optischen Kohärenzinterferometer, gegenüber anderen Analysesystemen, optische Grenzflächen und insbesondere eine Augenlänge besonders genau bestimmt werden können. So ist eine Messung einer Augenlänge mit einem Scheimpflugsystem mit einer vergleichbaren Genauigkeit nicht möglich.

Bei den bekannten OCT-Verfahren ist es nachteilig, dass die Gewinnung einer Bildaufnahme nicht unmittelbar erfolgen kann, d. h. dass ein entsprechender Zeitraum zum Scannen des aufzunehmenden Augenabschnitts mittels des Messtrahls erforderlich ist. Soll ein Tiefenscan erhalten werden, ist darüber hinaus eine Verstellung des Referenzarms mit dem entsprechenden Zeitaufwand erforderlich. Beim "time domain"- bzw. "frequency domain"-Verfahren ist eine sequentielle Aufnahme bzw. eine spektrale Durchstimmung einer Lichtquelle des Kohärenzinterferometers ggf. erforderlich, was ebenfalls eine zeitgleiche Aufnahme eines Augenabschnitts verhindert. So können Augenbewegungen im Zeitabschnitt der Bildaufnahme mit dem optischen Kohärenzinterferometer zu einer Verfälschung der Messergebnisse führen. Aufgrund der konvexen Oberfläche der Hornhaut des Auges kommt es bei einem Versatz des Messstrahls quer zum Auge in einer X-Achse ebenfalls zu einer Abstandsänderung in Richtung einer Z-Achse und ggf. zu einem Versatz in einer Y-Achse. Die Bewegung des Auges bewirkt folglich eine Krümmungsänderung der Hornhautoberfläche relativ zum Messstrahl sowie ggf. eine Änderung eines Brechungsindexes im Untersuchungsbereich des Auges. Insbesondere durch die Krümmungsänderung der Hornhaut ergibt sich ein weiterer Messfehler, da der Messstrahl beispielsweise an der Hornhautoberfläche durch die Bewegung des Auges und die dadurch bewirkte Krümmungsänderung abweichend abgelenkt wird. Zwar können Augenbewegungen durch einen Versatz eines empfangenen Signals erkannt werden, dies ermöglicht jedoch keine Korrektur eines aus einer Krümmungsänderung resultierenden Messfehlers.

Bei einer Bildaufnahme eines Augenabschnitts mit einem Scheimpflugsystem tritt das Problem von Messfehlern infolge von Augenbewegungen kaum auf, da das gesamte Schnittbild, entgegen einem gescannten Bild, im Wesentlichen zeitgleich aufgenommen wird, da eine Belichtungszeit eines Kamerachips vergleichsweise kurz ist. Falls dennoch innerhalb eines Belichtungszeitraums eine vergleichsweise schnelle Augenbewegung erfolgt, erscheint ein aufgenommenes Schnittbild unscharf. Dies kann jedoch aufgrund der zeitgleichen und vergleichsweise schnellen Bildaufnahme bei einer Messung regelmäßig verhindert werden.

Aus der DE 10 2010 046 500 A1 ist ein Kombinationsmessgerät bekannt, welches aus einem interferometrischen Messsystem und aus einem Scheimpflugsystem gebildet ist. Mit dem interferometrischen Messsystem zur optischen Kohärenztomographie (OCT) soll insbesondere ein sogenannter Ganzaugenscan zur tomographischen Abbildung eines gesamten Auges möglich sein. Die entsprechenden OCT-Daten können mit weiteren Messungen, insbesondere Scheimpflugaufnahmen, überlagert werden. Bei der Überlagerung der entsprechenden Bilddatensätze soll eine maßstabgetreue Verknüpfung erfolgen.

Die US 2010/0271594 A1 beschreibt ebenfalls ein Kombinationsgerät mit einem Scheimpflugsystem und einem OCT-System sowie ein entsprechendes Verfahren. Insbesondere wird eine Achslängenmessung des OCT-Systems durch eine nähere Bestimmung einer Augenvorderkammer durch das Scheimpflugsystem korrigiert.

Auch die US 2010/0014051 A1 offenbart ein entsprechendes Kombinationsmessgerät, wobei hier eine simultane Bestimmung einer Achslänge eines Auges durch eine Scheimpflugmessung und eine Interferometermessung vorgesehen sind.

Aus der WO 2014/028058 A1 ist ein Messverfahren bekannt, bei dem ein OCT-Bilddatensatz mit einem Scheimpflug-Bilddatensatz kombiniert wird. Dabei kann eine Bestimmung optischer Grenzflächen mit einem OCT-System genauer durchgeführt werden, als mit einem Scheimpflugsystem.

Die EP 2 281 500 A1 beschreibt ein ophthalmologisches Messverfahren beziehungsweise eine Vorrichtung, die ein Scheimpflugsystem zur Gewinnung von Schnittbildern eines Auges und ein optisches Kohärenzinterferometer kombiniert. Mit dem Scheimpflugsystem soll eine geometrische Referenz im Auge bestimmt werden, wobei nach der geometrischen Referenz die mit dem optischen Kohärenzinterferometer erfassten Detailstrukturen des Auges relativ zur Referenz positioniert werden. Ein Schnittbild des Scheimpflugsystems liegt dabei in einer übereinstimmenden Ebene mit einem Scan des optischen Kohärenzinterferometers.

Der vorliegenden Erfindung liegt daher die Aufgabe zugrunde, ein Verfahren und eine Vorrichtung zur Messung eines Auges vorzuschlagen, mit dem bzw. der eine einfache und schnelle besonders hochauflösende Bildaufnahme eines Bereiches des Auges ermöglicht wird.

Diese Aufgabe wird durch ein Verfahren mit den Merkmalen des Anspruchs 1 und ein Analysesystem mit den Merkmalen des Anspruchs 12 gelöst.

Das erfindungsgemäße Verfahren zur Untersuchung bzw. Messung einer Augengeometrie eines menschlichen Auges, insbesondere im Bereich eines vorderen Augenabschnitts eines Auges, wird mit einem ophthalmologischen Analysesystem durchgeführt, wobei das ophthalmologische Analysesystem ein erstes Analysesystem zur Gewinnung von Schnittbildern des Auges umfasst, wobei das erste Analysesystem aus einer Projektionseinrichtung und einer Beobachtungseinrichtung gebildet ist, die nach der Scheimpflugregel relativ zueinander angeordnet sind, wobei das ophthalmologische Analysesystem ein zweites Analysesystem zur Gewinnung von Schnittbildern des Auges umfasst, wobei das zweite Analysesystem aus einem optischen Kohärenzinterferometer gebildet ist, wobei das ophthalmologische Analysesystem eine Verarbeitungseinrichtung umfasst, wobei mit dem ersten Analysesystem ein erster Bilddatensatz und mit dem zweiten Analysesystem ein zweiter Bilddatensatz aus zumindest einer übereinstimmenden Aufnahmeebene des Auges gewonnen wird, wobei die Verarbeitungseinrichtung den ersten und den zweiten Bilddatensatz verarbeitet, wobei mittels der Verarbeitungseinrichtung der erste Bilddatensatz mit Daten des zweiten Bilddatensatzes zumindest teilweise ergänzt wird, wobei das zweite Analysesystem eine Strahlenlenkungseinrichtung umfasst, wobei die Strahlenlenkungseinrichtung einen bewegbaren Kippspiegel aufweist, der in einer Messachse des Analysesystems angeordnet ist, und mittels dem ein Messstrahl des optischen Kohärenzinterferometers auf die Hornhaut projiziert wird, wobei der Messstrahl innerhalb der Aufnahmeebene so abgelenkt wird, dass das Auge entlang der Aufnahmeebene durch den Messstrahl abgetastet wird, wobei die Strahlenlenkungseinrichtung einen Hohlspiegel aufweist, mittels dem ein Strahlengang bzw. Messstrahl des zweiten Analysesystems auf eine Hornhaut umgelenkt wird, wobei der Hohlspiegel so ausgebildet ist, dass eine optische Weglänge des Strahlengangs bis zur Hornhaut bei einer Abtastung verschiedener Punkte der Hornhaut im Wesentlichen unverändert lang ist.

Insbesondere dadurch, dass der erste Bilddatensatz, welcher mit dem ersten Analysesystem bzw. einem Scheimpflugsystem aufgenommen wurde, zumindest teilweise mit dem zweiten Bilddatensatz des optischen Kohärenzinterferometers ergänzt bzw. überlagert oder kombiniert wird, kann eine wesentliche Verbesserung einer Informationsdichte bzw. - auflösung des ersten Bilddatensatzes in einem ergänzten Teilbereich des ersten Bilddatensatzes erzielt werden. Da es nur noch erforderlich ist, in einem für eine spezifische Augenuntersuchung wesentlichen, begrenzten Bereich des ersten Bilddatensatzes bzw. der ersten Bildaufnahme detailliertere Bildinformationen zu erhalten, ist es ausreichend, wenn der erste Bilddatensatz in nur dem Teilbereich mit dem zweiten Bilddatensatz ergänzt wird. Der zweite Bilddatensatz kann dann auch mit dem optischen Kohärenzinterferometer besonders schnell durch Scannen eines Schnittbildes in der übereinstimmenden Aufnahmeebene des Auges aufgenommen werden, wodurch eine Gefahr einer eventuellen Bewegung des Auges während der Aufnahme des zweiten Bilddatensatzes erheblich gemindert wird. Insgesamt wird es so möglich, mit der Bildaufnahme eines Schnittbildes durch das Scheimpflugsystem bzw. das erste Analysesystem einen vergleichsweise großen Bereich eines Auges, wie einen vorderen Augenabschnitt, zu erfassen und mit dem optischen Kohärenzinterferometer einen ausgewählten Teilbereich des Bereichs mit hoher Auflösung und detailliert darzustellen und so potenzielle Messfehler durch Augenbewegungen zu vermeiden. Die Ergänzung des ersten Bilddatensatzes mit den Daten des zweiten Bilddatensatzes erfolgt mittels der Verarbeitungseinrichtung bzw. Mitteln zur Datenverarbeitung, mittels derer beide Bilddatensätze zu einem Ergebnisbilddatensatz kombiniert werden. Eine geometrische Auswertung des Ergebnisbilddatensatzes sowie dessen Bilddarstellung erfolgt mit dem bekannten Verfahren zur Bilddatenverarbeitung und - anzeige.

Das zweite Analysesystem umfasst neben dem optischen Kohärenzinterferometer eine Strahlenlenkungseinrichtung mit einem Kippspiegel mittels dem ein Messstrahl des optischen Kohärenzinterferometers auf die Hornhaut projiziert wird, wobei der Messstrahl innerhalb bzw. in der Aufnahmeebene so abgelenkt wird, dass das Auge entlang der Aufnahmeebene zur Gewinnung eines zweiten Bilddatensatzes durch den Messstrahl abgetastet wird. Folglich kann von der Strahlenlenkungseinrichtung ein Strahlengang des zweiten Analysesystems als Messstrahl ab- oder umgelenkt werden, so dass der Messstrahl in der Aufnahmeebene liegt. Zur Aufnahme eines Schnittbildes wird dann der Messstrahl durch die Aufnahmeebene hindurch bewegt bzw. entlang einer Linie bewegt, in der die Aufnahmeebene eine Hornhaut eines Auges schneidet. Beispielsweise können zur Gewinnung eines Schnittbildes mittels des optischen Kohärenzinterferometers dann je Schnittbild mindestens 4000 Messpunkte mit einer lateralen Auflösung von ca. 4 µm gemessen werden.

Die Strahlenlenkungseinrichtung weist den bewegbaren Kippspiegel auf, welcher zwischen dem zu untersuchenden Auge und dem zweiten Analysesystem angeordnet ist, wobei der Kippspiegel in einer Messachse des Analysesystems angeordnet ist. Mit dem Kippspiegel ist es dann möglich, einen relativ zum Auge verlaufenden Strahlengang des zweiten Analysesystems als Messstrahl abzulenken. Vorzugsweise kann das zweite Analysesystem so ausgebildet sein, dass der Strahlengang in Richtung einer Sehachse des Auges verläuft und auf den Kippspiegel trifft, der den Messstrahl dann ablenkt. Da zur Erfassung eines Schnittbildes lediglich ein linienförmiger Scan des Auges mittels des Messstrahls erforderlich ist, wird alleine nur ein Kippspiegel benötigt. Eine Erfassung eines Schnittbildes bzw. eines zweiten Bilddatensatzes kann beispielsweise innerhalb 20 bis 40 Millisekunden erfolgen. Dann ist es auch möglich, den zweiten Bilddatensatz im Wesentlichen zeitgleich mit dem ersten Bilddatensatz aufzunehmen.

Die Strahlenlenkungseinrichtung weist einen Hohlspiegel auf, der in Richtung eines Strahlengangs des zweiten Analysesystems nachfolgend einem Kippspiegel angeordnet ist und mittels dem der Strahlengang bzw. ein Messstrahl des zweiten Analysesystems auf eine Hornhaut eines zu untersuchenden Auges umgelenkt wird, wobei der Hohlspiegel so ausgebildet ist, dass eine optische Weglänge des Strahlengangs des zweiten Analysesystems bzw. eines Messarmes des optischen Kohärenzinterferometers bis zur Hornhaut bei einer Abtastung verschiedener Punkte der Hornhaut im Wesentlichen unverändert ist. Demnach wird der von dem Kippspiegel abgelenkte Messstrahl auf den asphärischen Hohlspiegel umgelenkt und dann von dem asphärischen Hohlspiegel auf die Hornhaut des Auges abgelenkt. Durch den Hohlspiegel bzw. asphärischen Hohlspiegel wird es dann möglich, die Länge des Messarms bzw. des zugehörigen Strahlengangs des optischen Kohärenzinterferometers relativ zu einer Oberfläche der Hornhaut im Wesentlichen konstant bzw. gleich zu halten. Ein axialer Messbereich bzw. eine Messtiefe des optischen Kohärenzinterferometers kann so im Wesentlichen der Oberfläche der Hornhaut angepasst werden.

In einer Ausführungsform des Verfahrens kann mittels des zweiten Analysesystems alleine eine Bildaufnahme einer Hornhaut des Auges mit einem "frequency domain"-Verfahren erfolgen. Ein Vorteil des "frequency domain"-Verfahrens liegt in einer einfachen und schnellen simultanen Messung, wobei vollständige Informationen über eine Tiefe der Messung ermittelt werden können, ohne dass ein beweglicher Referenzarm ermöglicht wird. Um eine Messgeschwindigkeit noch weiter zu erhöhen, kann ein "spectral domain"-Verfahren angewendet werden, wobei eine spektrale Information eines gewonnenen Signals mittels eines Spektrometers parallel, d. h. zeitgleich, erfasst wird. Ein spectral domain OCT-System ist vergleichweise einfach aufgebaut und kostengünstig zu erhalten. Weiter kann dann bis zu einer Tiefe von mindestens 3 mm in der Aufnahmeebene des Auges gemessen werden.

Weiter kann mittels des zweiten Analysesystems zur Gewinnung einer Bildaufnahme eine Tiefenprofilaufnahme einer Hornhaut des Auges erfolgen. Mittels eines sogenannten amplitude-mode scans bzw. A-Scans kann eine zweidimensionale Bildaufnahme bzw. ein Schnittbild aus der Aufnahmeebene des Auges gewonnen werden. Ein A-Scan ist gegenüber einem B- oder C-Scan besonders schnell durchzuführen und erfolgt durch Abtasten bzw. Scannen des Auges längs der Aufnahmeebene. Dennoch können in Abwandlungen des Verfahrens auch B- und C-Scans durchgeführt werden.

Besonders vorteilhaft ist es, wenn der erste und der zweite Bilddatensatz im Wesentlichen gleichzeitig erfasst bzw. aufgenommen werden. So kann vermieden werden, dass zwischen der ersten Bildaufnahme und der zweiten Bildaufnahme eine Augenbewegung erfolgen kann. Durch die zeitgleiche Aufnahme des ersten und zweiten Bilddatensatzes werden so mögliche Fehlerquellen bei einer Messung ausgeschlossen.

Weiter kann vor der Ergänzung des ersten Bilddatensatzes eine Korrektur des zweiten Bilddatensatzes nach dem ersten Bilddatensatz erfolgen. Der erste Bilddatensatz kann dann als ein Referenzbilddatensatz herangezogen werden, nachdem der zweite Bilddatensatz relativ zum ersten Bilddatensatz positioniert wird. Weiter können eventuelle Aufnahmefehler im zweiten Bilddatensatz, die durch eine Augenbewegung im Aufnahmezeitraum des zweiten Bilddatendatzes bewirkt sein können, dahingehend korrigiert werden, dass unter Einberechnung einer aus dem ersten Bilddatensatz bekannten Krümmung einer Hornhaut der zweite Bilddatensatz zumindest teilweise neu berechnet wird. Eine Krümmungsänderung der Hornhaut durch eine Augenbewegung während eines Scans mit dem optischen Kohärenzinterferometer kann dann zur Neuberechnung der nach der Augenbewegung gescannten Teilbereiche des Auges herangezogen werden.

Auch kann der zweite Bilddatensatz nach aus dem ersten Bilddatensatz ermittelten optischen Grenzflächen, beispielsweise durch einen Vergleich beider Bilddatensätze, ausgerichtet bzw. korrigiert werden. Die optischen Grenzflächen, die im ersten Bilddatensatz enthalten sind, eignen sich besonders gut zur Ausrichtung bzw. zur Korrektur des zweiten Bilddatensatzes, sofern dieser durch eine Augenbewegung verfälscht wurde. Auch ist es so möglich, den zweiten Bilddatensatz nach dem ersten Bilddatensatz zu skalieren, sofern der zweite Bilddatensatz verzerrt sein oder aber einen anderen Maßstab aufweisen sollte. Mittels des ersten Analysesystems können Brechungsindizes und/oder eine Topografie einer Hornhaut ermittelt werden, die bei der Korrektur des zweiten Bilddatensatzes berücksichtigt werden können. So wird es möglich, eine noch genauere Korrektur des zweiten Bilddatensatzes durchzuführen.

Vorzugsweise kann der erste Bilddatensatz mit dem zweiten Bilddatensatz überlagert werden. So wird es möglich, weniger detaillierte Darstellungen eines Teilbereichs des ersten Bilddatensatzes mit detaillierteren Darstellungen des zweiten Bilddatensatzes derart zu überlagern, dass der betreffende Bereich des ersten Bilddatensatzes überdeckt oder alternativ durch additive Bildverarbeitung neu berechnet wird.

Um einen dreidimensionalen Bilddatensatz von einem Auge zu erhalten, kann eine Mehrzahl erster und zweiter Bilddatensätze in einer sequenziellen Abfolge gewonnen werden, wobei die gemeinsame Aufnahmeebene des ersten und zweiten Analysesystems um eine Sehachse des Auges verschwenkt werden kann. Die in der sequenziellen Abfolge aufgenommenen Schnittbilder werden mittels der Verarbeitungseinrichtung dann zu einem dreidimensionalen Modell des Auges zusammengesetzt. Die Rotation des ersten und des zweiten Analysesystems um die Sehachse kann zumindest um 180 ° erfolgen, um so die Aufnahmeebene gleichmäßig über dem Bereich des vorderen Augenabschnitts zu verschwenken. Die Aufnahmeebene kann demnach axial um die Sehachse rotiert werden, wobei bis zu 100 Schnittbilder mit jeweils dem ersten und dem zweiten Analysesystem nacheinander in verschiedenen Winkeln α relativ zu einer horizontalen Ebene aufgenommen werden können.

Vorteilhaft ist es auch, wenn eine Relativposition zumindest einer mit dem ersten Analysesystem ermittelten optischen Grenzfläche des Auges als eine Referenzfläche oder ein Referenzpunkt für das zweite Analysesystem bestimmt wird. Mittels des ersten Analysesystems kann eine relative Lage des zu untersuchenden Auges relativ zum Analysesystem besondes genau bestimmt werden. So können auch Augenbewegungen während einer Aufnahme einer Sequenz von Schnittbildern leicht erkannt werden. Ein mit dem zweiten Analysesystem gewonnenes Schnittbild kann dann besonders einfach nach dem zugehörigen Schnittbild des ersten Analysesystems bzw. der zugehörigen Schnittbildaufnahme der Sequenz des ersten Analysesystems räumlich, bezogen auf das Auge, korrekt angeordnet werden. Als eine Referenzfläche bzw. -linie oder -punkt kann eine optische Grenzfläche des Auges, wie z. B. die Hornhautoberfläche, herangezogen werden.

Das erste Analysesystem und das zweite Analysesystem können jeweils Licht bzw. elektromagnetische Strahlung mit voneinander verschiedenen Wellenlängenbereichen aussenden. Dadurch kann der Vorteil erzielt werden, dass die jeweiligen Strahlengänge deckungsgleich sein können, wobei eine Trennung der Strahlengänge bzw. eine voneinander getrennte Bilderfassung über geeignete optische Filter erfolgen kann. Beispielsweise kann für das optische Kohärenzinterferometer Licht mit einer Wellenlänge von 800 nm vorteilhaft verwendet werden, da dann im Handel kostengünstig erhältliche Infrarotsensoren eingesetzt werden können. Die Projektionseinrichtung des ersten Analysesystems kann beispielsweise Licht einer Wellenlänge von 475 nm aussenden. Im Übrigen wird so auch weitestgehend eine Blendung einer zu untersuchenden Person vermieden.

Das erfindungsgemäße ophthalmologische Analysesystem zur Untersuchung bzw. Messung einer Augengeometrie eines Auges, insbesondere im Bereich eines vorderen Augenabschnitts eines Auges, umfasst ein erstes Analysesystem zur Gewinnung von Schnittbildern des Auges, wobei das erste Analysesystem aus einer Projektionseinrichtung und einer Beobachtungseinrichtung gebildet ist, die nach der Scheimpflugregel relativ zueinander angeordnet sind, und umfasst weiter ein zweites Analysesystem zur Gewinnung von Schnittbildern des Auges, wobei das zweite Analysesystem aus einem optischen Kohärenzinterferometer gebildet ist, wobei das ophthalmologische Analysesystem eine Verarbeitungseinrichtung umfasst, wobei mit dem ersten Analysesystem ein erster Bilddatensatz und mit dem zweiten Analysesystem ein zweiter Bilddatensatz aus zumindest einer übereinstimmenden Aufnahmeebene des Auges gewinnbar ist, wobei die Verarbeitungseinrichtung so ausgebildet ist, dass diese den ersten und den zweiten Bilddatensatz verarbeiten kann, wobei mittels der Verarbeitungseinrichtung der erste Bilddatensatz mit Daten des zweiten Bilddatensatzes zumindest teilweise ergänzbar ist, wobei das zweite Analysesystem eine Strahlenlenkungseinrichtung umfasst, wobei die Strahlenlenkungseinrichtung einen bewegbaren Kippspiegel aufweist, der in einer Messachse des Analysesystems angeordnet ist, und mittels dem ein Messstrahl des optischen Kohärenzinterferometers auf die Hornhaut projiziert werden kann, wobei der Messstrahl innerhalb der Aufnahmeebene so abgelenkt werden kann, dass das Auge entlang der Aufnahmeebene durch den Messstrahl abgetastet werden kann, wobei die Strahlenlenkungseinrichtung einen Hohlspiegel aufweist, mittels dem ein Strahlengang bzw. Messstrahl des zweiten Analysesystems auf eine Hornhaut umgelenkt werden kann, wobei der Hohlspiegel so ausgebildet ist, dass eine optische Weglänge des Strahlengangs bis zur Hornhaut bei einer Abtastung verschiedener Punkte der Hornhaut im Wesentlichen unverändert lang ist. Die Vorteile des erfindungsgemäßen ophthalmologischen Analysesystems betreffend wird auf die Vorteilsbeschreibung des erfindungsgemäßen Verfahrens verwiesen.

Weiter kann die Strahlenlenkungseinrichtung so ausgebildet sein, dass der Messstrahl auf einen Oberflächenpunkt einer Hornhaut projiziert werden kann, wobei der Messstrahl relativ zu einer Tangentialebene des Oberflächenpunktes, unter einem von 90 ° abweichenden Winkel, auf die Hornhaut projiziert werden kann. Der Oberflächenpunkt entspricht demnach dem Punkt, in dem der Messstrahl auf die Hornhaut auftrifft. Da der Messstrahl dann relativ zu der Tangentialebene und nicht senkrecht auf die Hornhaut auftrifft, erfolgt keine direkte Reflektion des Messstrahls von der Oberfläche der Hornhaut. Eine direkte Reflektion ist sehr hell und kann zu Überstrahlungen bei einer Detektion des Reflektionsstrahls führen, die nur sehr schwer zu korrigieren sind. Derartige Überstrahlungen können so wirkungsvoll vermieden werden.

Das ophthalmologische Analysesystem ist besonders einfach ausbildbar, wenn Strahlengänge des ersten und des zweiten Analysesystems gemeinsam auf der Messachse verlaufen, und wenn dann der Kippspiegel ein dichroitischer Spiegel ist. Mit dem dichroitischen Spiegel ist es dann möglich, die jeweiligen Strahlengänge zu trennen. Beispielsweise kann der dichroitische Spiegel so ausgebildet sein, dass nur Licht des zweiten Analysesystems reflektiert wird. Licht des ersten Analysesystems kann dann durch den Kippspiegel unreflektiert hindurchtreten.

Ein Messbereich des zweiten Analysesystems kann an eine Krümmung einer Hornhaut angepasst sein. Dabei ist es zunächst unerheblich, wie die Krümmungsanpassung erfolgt. Je nach Gestalt des Hohlspiegels kann der Messbereich in der Aufnahmeebene eine Tiefe von zumindest 3 mm aufweisen und bogenförmig, an die Querschnittsform der Hornhaut angepasst, ausgebildet sein. Bei einer derartigen Ausgestaltung des ophthalmologischen Analysesystems kann ein Messabstand zu einem zu untersuchenden Auge beispielsweise ca. 80 mm betragen.

Um Bilddatensätze zu erhalten, die eine dreidimensionale Reproduktion eines Auges ermöglichen, kann die gemeinsame Aufnahmeebene des ersten und zweiten Analysesystems um eine Sehachse des Auges mittels einer Rotationseinrichtung verschenkbar sein, wobei das optische Kohärenzinterferometer dann eine optische Faser bzw. Lichtleitfaser aufweisen kann, die mechanisch von der Rotationseinrichtung entkoppelt ist. Die Rotationseinrichtung kann beispielsweise durch das erste Analysesystem ausgebildet werden, wobei eine Kamera in einer Scheimpfluganordnung um die Sehachse bzw. eine Messachse des Auges zusammen mit der Projektionseinrichtung bzw. einer Spaltbeleuchtung rotiert werden kann. Zeitgleich kann eine Rotation eines Messstrahls des optischen Kohärenzinterferometers mittels einer Strahlenlenkungseinrichtung relativ zur Aufnahmeebene erfolgen. Wenn ein Strahlengang des optischen Kohärenzinterferometers der rotierenden Strahlenlenkungseinrichtung über eine Lichtleitfaser zugeführt wird, ist es ggf. erforderlich, die Lichtleitfaser von der Drehung der Strahlenlenkungseinrichtung bzw. der Rotationseinrichtung mechanisch zu entkoppeln. Dies ist insbesondere dann erforderlich, wenn eine vollständig freie Drehung der Rotationseinrichtung möglich ist. Weiter kann ein Spektrometer des optischen Kohärenzinterferometers auch innerhalb eines Gerätegehäuses des ersten Analysesystems integriert sein. Darüber hinaus ist es möglich, das erste und das zweite Analysesystem in einem gemeinsamen Gehäuse auszubilden.

Weitere vorteilhafte Ausführungsformen des ophthalmologischen Analysesystems ergeben sich aus den Merkmalsbeschreibungen der auf das erfindungsgemäße Verfahren rückbezogenen Unteransprüche.

Nachfolgend werden bevorzugte Ausführungsformen des Verfahrens und des ophthalmologischen Analysesystems anhand der Zeichnungen näher erläutert.

Es zeigen:
- **Fig. 1**: Eine schematische Ansicht eines vorderen Augenabschnitts zusammen mit einer Ausführungsform einer Strahlenlenkungseinrichtung;
- **Fig. 2**: eine Vorderansicht eines Auges in Richtung einer Sehachse;
- **Fig. 3**: eine Schnittansicht des Auges in einer Aufnahmeebene;
- **Fig. 4**: eine schematische Teildarstellung eines Geräteaufbaus zusammen mit einer Teilschnittansicht des Auges in einer Seitenansicht;
- **Fig. 5**: die schematische Teildarstellung aus **Fig. 4** in einer Draufsicht.

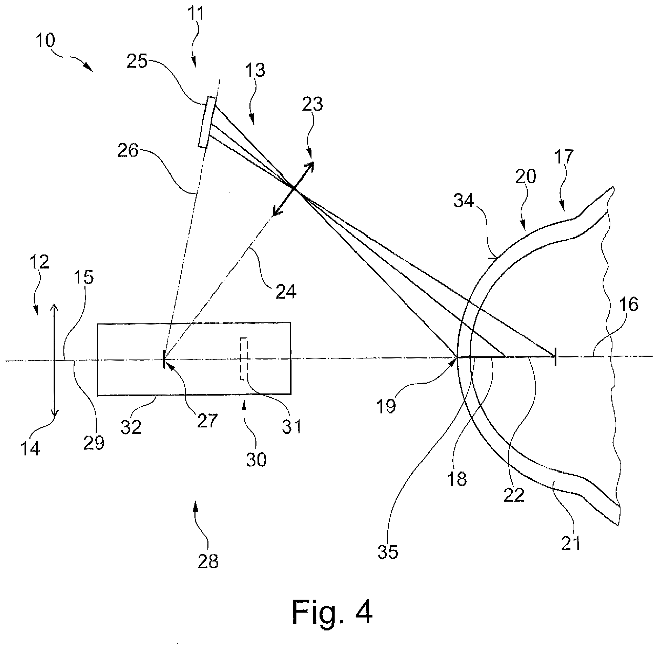

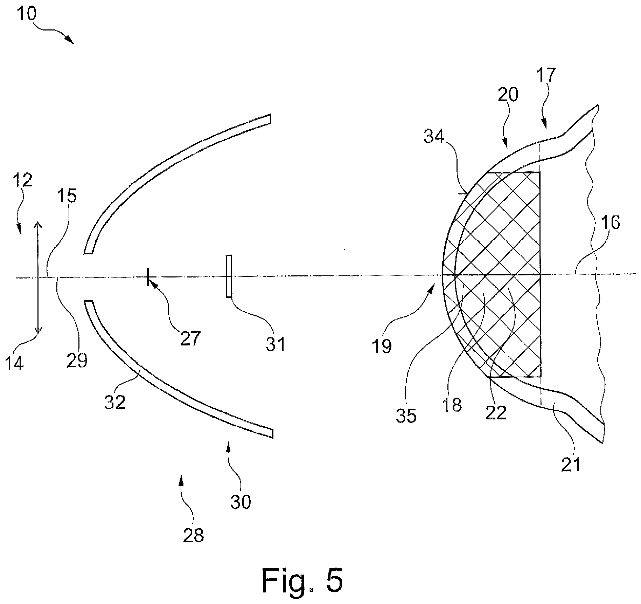

Den **Fig. 4** und **5** ist der prinzipielle Aufbau eines ophthalmologischen Analysesystems 10 zu entnehmen. Ein hier teilweise dargestelltes erstes Analysesystem 11 des ophthalmologischen Analysesystems 10 ist aus einer Projektionseinrichtung 12 und einer Beobachtungseinrichtung 13 gebildet. Von der Projektionseinrichtung 12 ist hier lediglich ein Objektiv 14 schematisch angedeutet, durch das ein Strahlengang 15 einer hier nicht näher dargestellten Spaltbeleuchtung entlang einer Sehachse 16 eines Auges 17 auf das Auge 17 projiziert wird. In den **Fig. 4** und **5** verläuft der Strahlengang 15 der Spaltbeleuchtung in einer Aufnahmeebene 18, so dass die Spaltbeleuchtung bzw. die Aufnahmeebene 18 hier horizontal und orthogonal relativ zu einer Ansichtsebene der **Fig. 4** bzw. parallel zu einer Ansichtsebene der **Fig. 5** verläuft. Der Strahlengang 15 tritt im Bereich eines Apex 19 des Auges 17 in einen vorderen Augenabschnitt 20 mit einer hier schematisch dargestellten Hornhaut 21 des Auges 17 ein und erzeugt aufgrund einer Lichtstreuung innerhalb des Auges 17 ein Schnittbild 22, das in der **Fig. 5** schraffiert dargestellt ist. Das Schnittbild 22 wird mittels der Beobachtungseinrichtung 13, welche aus einem Objektiv 23 mit einer Hauptebene 24 und einem Kamerachip 25 gebildet ist, aufgenommen. Eine Bildebene 26 des Kamerachips 25 sowie die Hauptebene 24 sind so ausgerichtet, dass sie sich mit der Aufnahmeebene 18 in einem Punkt 27 schneiden, so dass die Scheimpflugregel für die Beobachtungseinrichtung 13 erfüllt ist. Das Schnittbild 22 wird folglich auf dem Kamerachip 25 unverzerrt abgebildet. Ein zweites Analysesystem 28 des ophthalmologischen Analysesystems 10 ist aus einem hier nicht näher dargestellten optischen Kohärenzinterferometer gebildet, welches das Objektiv 14 der Beobachtungseinrichtung 13 nutzt. Ein Strahlengang 29 des zweiten Analysesystems 28 ist deckungsgleich mit dem Strahlengang 15 der Projektionseinrichtung 12 bzw. der Sehachse 16 und repräsentiert einen Messarm des optischen Kohärenzinterferometers. Weiter umfasst das zweite Analysesystem eine Strahlenlenkungseinrichtung 30 mit einem dichroitischen Kippspiegel 31 und einem asphärischen Hohlspiegel 32. Der asphärische Hohlspiegel 32 ist zweiteilig ausgebildet.

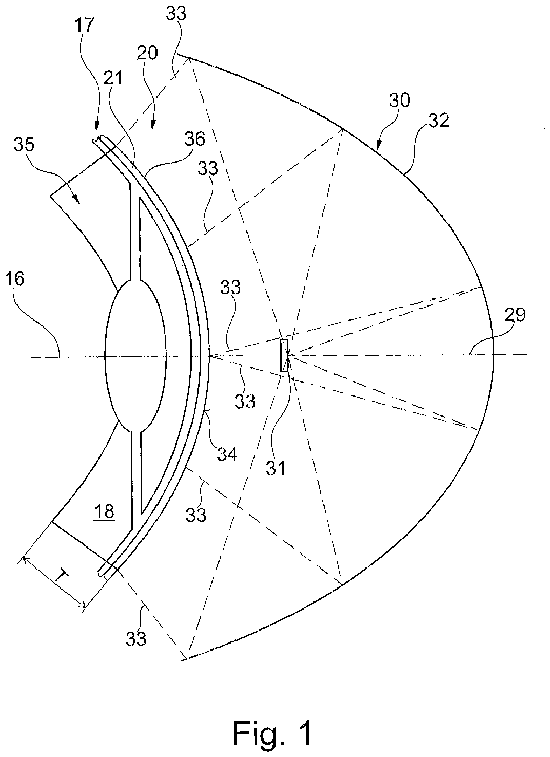

Die in **Fig. 1** gezeigte schematische Ansicht entspricht einer Draufsicht auf die in **Fig. 4** horizontal verlaufende Aufnahmeebene 18.

Wie aus einer Zusammenschau der **Fig. 1** und **4** zu erkennen ist, wird der Strahlengang 29 des zweiten Analysesystems 28 nach dem Durchtritt durch das Objektiv 14 von dem Kippspiegel 31 innerhalb der Aufnahmeebene 18 reflektiert und auf den asphärischen Hohlspiegel 32 projiziert. Der asphärische Hohlspiegel 32 reflektiert den Strahlengang 29 auf die Hornhaut 21 des Auges 17. Nach der **Fig. 1** kann der Strahlengang 29 über dem Kippspiegel 39 als ein Messstrahl 33, wie aus **Fig. 1** zu ersehen ist, in einer Vielzahl von Richtungen innerhalb der Aufnaheebene 18 abgelenkt werden und bis zu einer Messtiefe T von mindestens 3 mm in das Auge 17 eindringen. Dabei wird mit dem optischen Kohärenzinterferometer ein spectral-domain-Scan durchgeführt, bei dem eine axiale Auflösung bzw. Tiefeneinlösung von 1 bis 5 µm erzielt wird, und wobei mindestens 4000 Messpunkte entlang der Hornhaut 21 in der Aufnahmeebene 18 aufgenommen werden.

Weiter ist vorgesehen, dass der Strahlengang 15 und der Strahlengang 29 jeweils aus Licht unterschiedlicher Wellenlänge ausgebildet ist, so dass lediglich der Strahlengang 29 von dem dichroitischen Kippspiegel 31 reflektiert wird und der Strahlengang 15 den dichroitischen Kippspiegel 31 ungehindert durchdringen kann.

Durch das Zusammenwirken des Kippspiegels 31 und des asphärischen Hohlspiegels 32 wird eine Länge des Strahlengangs 29 bzw. des Messstrahls 33 relativ bezogen auf eine Oberfläche 34 der Hornhaut 21 angepasst, so dass ein Schnittbild 35 des zweiten Analysesystems 28 an eine Kontur 36 der Oberfläche 34 der Hornhaut 21 angepasst ist und im Wesentlichen bogenförmig verläuft. Damit kann ein optisches Kohärenzinterferometer mit einem starren, nicht verschieblichen Referenzarm verwendet und ein Messbereich des zweiten Analysesystems optimal an das zu messende Auge 17 angepasst werden.

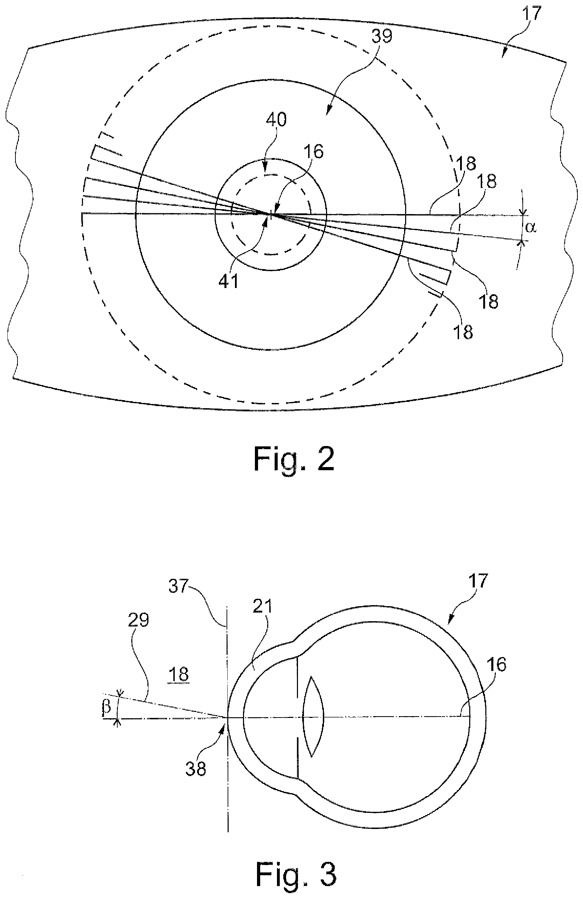

Um eine direkte Reflektion des Messstrahls 33, und damit verbunden Überblendungen an einem Detektor eines Spektrometers des optischen Kohärenzinterferometers, zu vermeiden, wird der Messstrahl 33, wie aus der schematischen Darstellung in **Fig. 3** zu ersehen ist, relativ zu einer Tangentialebene 37 eines Oberflächenpunktes 38 der Hornhaut 21, der von dem Messstrahl 33 getroffen wird, in einem von 90 ° abweichenden Winkel β auf die Hornhaut 21 projiziert. Die **Fig. 3** zeigt hier eine Draufsicht auf die Aufnahmeebene 18.

Die **Fig. 2** zeigt eine schematische Vorderansicht des Auges 17 mit einer Iris 39 und einer Pupille 40 sowie einer Rotationsachse 41 auf der Sehachse 16. Die Projektionseinrichtung 12 bzw. die Spaltbeleuchtung mit der Beobachtungseinrichtung 13 ist zusammen mit dem Kippspiegel 31 nun um die Rotationsachse 41 so drehbar, dass die Aufnahmeebene 18 um einen Winkel α um die Sehachse 16 bzw. die Rotationsachse 41 verschwenkt werden kann. Durch eine Rotation bzw. Drehung von mindestens 180 ° und einer Aufnahme von jeweils Schnittbildern 22 und 35 mit dem ersten Analysesystem 11 und dem zweiten Analysesystem 28 in jeder Position der Aufnahmeebene 18 ist es dann möglich, eine Vielzahl von Schnittbildern 22 und 35 des Auges 17 zu erhalten, um diese mittels einer hier nicht gezeigten Verarbeitungseinrichtung des ophthalmologischen Analysesystems zu einem dreidimensionalen Bild des Auges 17 bzw. dessen vorderen Augenabschnitt 20 zusammenzufügen. Dabei wird insbesondere jeweils für die Schnittbilder 22 und 35 jeder Aufnahmeebene 18 ein jeweils erster Bilddatensatz des ersten Analysesystems 11 mit Daten eines zweiten Bilddatensatzes des zweiten Analysesystems 28 zumindest teilweise ergänzt. So ist es dann möglich, das Schnittbild 22, welches gegenüber dem Schnittbild 35 eine vergleichsweise größere Fläche aufweist, mit Bilddaten des Schnittbildes 35 zu ergänzen und im Bereich des Schnittbildes 35 eine detailliertere Darstellung des Auges 17 innerhalb eines Teilbereiches des Schnittbildes 22 zu erzielen.

## Patentansprüche

1. Verfahren zur Untersuchung eines Auges, im Bereich eines vorderen Augenabschnitts (20) eines Auges (17), mit einem ophthalmologischen Analysesystem (10), umfassend ein erstes Analysesystem (11) zur Gewinnung von ersten Schnittbildern (22) des Auges,
wobei das erste Analysesystem aus einer Projektionseinrichtung (12) und einer Beobachtungseinrichtung (13) gebildet ist, die nach der Scheimpflugregel relativ zueinander angeordnet sind, und umfassend ein zweites Analysesystem (28) zur Gewinnung von zweiten Schnittbildern (35) des Auges, wobei das zweite Analysesystem aus einem optischen Kohärenzinterferometer gebildet ist, wobei das ophthalmologische Analysesystem eine Verarbeitungseinrichtung umfasst, wobei mit dem ersten Analysesystem ein erster Bilddatensatz und mit dem zweiten Analysesystem ein zweiter Bilddatensatz aus zumindest einer übereinstimmenden Aufnahmeebene (18) des Auges gewonnen wird, wobei die Verarbeitungseinrichtung den ersten und den zweiten Bilddatensatz verarbeitet, wobei mittels der Verarbeitungseinrichtung der erste Bilddatensatz mit Daten des zweiten Bilddatensatzes zumindest teilweise ergänzt wird, wobei
das zweite Analysesystem (28) eine Strahlenlenkungseinrichtung (30) umfasst, wobei die Strahlenlenkungseinrichtung (30) einen bewegbaren Kippspiegel (31) aufweist, der in einer Messachse des Analysesystems (10) angeordnet ist, und mittels dem ein Messstrahl (33) des optischen Kohärenzinterferometers auf die Hornhaut (21) projiziert wird, wobei der Messstrahl innerhalb der Aufnahmeebene (18) so abgelenkt wird, dass das Auge (17) entlang der Aufnahmeebene durch den Messstrahl abgetastet wird, **dadurch gekennzeichnet, dass** die Strahlenlenkungseinrichtung (30) einen Hohlspiegel (32) aufweist, mittels dem der Messstrahl (33) auf eine Hornhaut (21) umgelenkt wird, wobei der Hohlspiegel so ausgebildet ist, dass eine optische Weglänge des Messstrahls bis zur Hornhaut bei einer Abtastung verschiedener Punkte der Hornhaut unverändert lang ist.

2. Verfahren nach Anspruch 1,
**dadurch gekennzeichnet,**
**dass** mittels des zweiten Analysesystems (28) eine Bildaufnahme einer Hornhaut (21) des Auges (17) mit einem Frequenzdomänenverfahren erfolgt.

3. Verfahren nach Anspruch 1 oder 2,
**dadurch gekennzeichnet,**
**dass** mittels des zweiten Analysesystems (28) zur Gewinnung einer Bildaufnahme eine Tiefenprofilaufnahme einer Hornhaut (21) des Auges (17) erfolgt.

4. Verfahren nach einem der vorangehenden Ansprüche,
**dadurch gekennzeichnet,**
**dass** der erste und der zweite Bilddatensatz gleichzeitig erfasst werden.

5. Verfahren nach einem der vorangehenden Ansprüche,
**dadurch gekennzeichnet,**
**dass** vor der Ergänzung des ersten Bilddatensatzes eine Korrektur des zweiten Bilddatensatzes nach dem ersten Bilddatensatz erfolgt.

6. Verfahren nach Anspruch 5,
**dadurch gekennzeichnet,**
**dass** der zweite Bilddatensatz nach aus dem ersten Bilddatensatz ermittelten optischen Grenzflächen korrigiert wird.

7. Verfahren nach Anspruch 5 oder 6,
**dadurch gekennzeichnet,**
**dass** mittels des ersten Analysesystems (11) Brechungsindizes und/oder eine Topografie einer Hornhaut (21) ermittelt werden, die bei der Korrektur berücksichtigt werden.

8. Verfahren nach einem der vorangehenden Ansprüche,
**dadurch gekennzeichnet,**
**dass** der erste Bilddatensatz mit dem zweiten Bilddatensatz überlagert wird.

9. Verfahren nach einem der vorangehenden Ansprüche,
**dadurch gekennzeichnet,**
**dass** eine Mehrzahl erster und zweiter Bilddatensätze in einer sequentiellen Abfolge gewonnen wird, wobei die gemeinsame Aufnahmeebene (18) des ersten und zweiten Analysesystems (11, 28) um eine Sehachse (16) des Auges (17) verschwenkt wird.

10. Verfahren nach einem der vorangehenden Ansprüche,
**dadurch gekennzeichnet,**
**dass** eine Relativposition zumindest einer mit dem ersten Analysesystem (11) ermittelten optischen Grenzfläche des Auges (17) als eine Referenzfläche für das zweite Analysesystem (28) bestimmt wird.

11. Verfahren nach einem der vorangehenden Ansprüche,
**dadurch gekennzeichnet,**
**dass** das erste Analysesystem (11) und das zweite Analysesystem (28) jeweils Licht mit voneinander verschiedenen Wellenlängenbereichen aussenden.

12. Ophthalmologisches Analysesystem (10) zur Untersuchung eines Auges, im Bereich eines vorderen Augenabschnitts (20) eines Auges (17), umfassend ein erstes Analysesystem (11) zur Gewinnung von ersten Schnittbildern (22) des Auges, wobei das erste Analysesystem aus einer Projektionseinrichtung (12) und einer Beobachtungseinrichtung (13) gebildet ist, die nach der Scheimpflugregel relativ zueinander angeordnet sind, und umfassend ein zweites Analysesystem (28) zur Gewinnung von zweiten Schnittbildern (35) des Auges, wobei das zweite Analysesystem aus einem optischen Kohärenzinterferometer gebildet ist, wobei das ophthalmologische Analysesystem eine Verarbeitungseinrichtung umfasst, wobei mit dem ersten Analysesystem ein erster Bilddatensatz und mit dem zweiten Analysesystem ein zweiter Bilddatensatz aus zumindest einer übereinstimmenden Aufnahmeebene (18) des Auges gewinnbar ist, wobei die Verarbeitungseinrichtung so ausgebildet ist, dass diese den ersten und den zweiten Bilddatensatz verarbeiten kann, wobei mittels der Verarbeitungseinrichtung der erste Bilddatensatz mit Daten des zweiten Bilddatensatzes zumindest teilweise ergänzbar ist, wobei das zweite Analysesystem (28) eine Strahlenlenkungseinrichtung (30) umfasst, wobei die Strahlenlenkungseinrichtung (30) einen bewegbaren Kippspiegel (31) aufweist, der in einer Messachse des Analysesystems (10) angeordnet ist, und mittels dem ein Messstrahl (33) des optischen Kohärenzinterferometers auf die Hornhaut (21) projiziert werden kann, wobei der Messstrahl innerhalb der Aufnahmeebene (18) so abgelenkt werden kann, dass das Auge (17) entlang der Aufnahmeebene durch den Messstrahl abgetastet werden kann,
**dadurch gekennzeichnet,**
**dass** die Strahlenlenkungseinrichtung (30) einen Hohlspiegel (32) aufweist, mittels dem der Messstrahl (33) auf eine Hornhaut (21) umgelenkt werden kann, wobei der Hohlspiegel so ausgebildet ist, dass eine optische Weglänge des Messstrahls bis zur Hornhaut bei einer Abtastung verschiedener Punkte der Hornhaut unverändert lang ist.

13. Analysesystem nach Anspruch 12,
**dadurch gekennzeichnet,**
**dass** die Strahlenlenkungseinrichtung (30) so ausgebildet ist, dass der Messstrahl (33) auf einen Oberflächenpunkt (38) einer Hornhaut (21) projiziert werden kann, wobei der Messstrahl relativ zu einer Tangentialebene (37) des Oberflächenpunktes unter einem von 90 ° abweichenden Winkel auf die Hornhaut projiziert werden kann.

14. Analysesystem nach Anspruch 12 oder 13,
**dadurch gekennzeichnet,**
**dass** Strahlengänge (15, 29) des ersten und des zweiten Analysesystems (11, 28) gemeinsam auf der Messachse verlaufen, und wobei der Kippspiegel (31) ein dichroitischer Spiegel ist.

15. Analysesystem nach einem der Ansprüche 12 bis 14,
**dadurch gekennzeichnet,**
**dass** ein Messbereich des zweiten Analysesystems (28) an eine Krümmung einer Hornhaut (21) angepasst ist.

16. Analysesystem nach einem der Ansprüche 12 bis 15,
**dadurch gekennzeichnet,**
**dass** die gemeinsame Aufnahmeebene (18) des ersten und zweiten Analysesystems (11, 28) um eine Sehachse (16) des Auges (17) mittels einer Rotationseinrichtung verschwenkbar ist, wobei das optische Kohärenzinterferometer eine optische Faser aufweist, die mechanisch von der Rotationseinrichtung entkoppelt ist.

## Claims

1. A method for examining an eye in the region of a front eye section (20) of an eye (17), using an ophthalmological analysis system (10), comprising a first analysis system (11) for obtaining first sectional images (22) of the eye, wherein the first analysis system is formed from a projection device (12) and from a monitoring device (13), which are arranged relative to each other according to the Scheimpflug rule, and comprising a second analysis system (28) for obtaining second sectional images (35) of the eye, wherein the second analysis system is formed from an optical coherence interferometer, wherein the ophthalmological analysis system comprises a processing device, wherein, from at least one coinciding recording plane (18) of the eye, a first image data set is obtained using the first analysis system and a second image data set is obtained using the second analysis system, wherein the processing device processes the first and the second image data set, wherein, by means of the processing device, the first image data set is supplemented, at least partially, with data of the second image data set, wherein the second analysis system (28) comprises a beam guidance device (30), wherein the beam guidance device (30) has a movable tilting mirror (31) which is arranged in a measurement axis of the analysis system (10), and by means of which a measurement beam (33) of the optical coherence interferometer is projected onto the cornea (21), wherein the measurement beam is diverted within the recording plane (18) such that the eye (17) is scanned by the measurement beam along the recording plane,
**characterised in that**
the beam guidance device (30) has a concave mirror (32), by means of which the measurement beam (33) is deflected onto a cornea (21), wherein the concave mirror is constructed such that an optical path length of the measurement beam up to the cornea remains unchanged when scanning different points of the cornea.

2. The method according to claim 1,
**characterised in that**
by means of the second analysis system (28), an image of a cornea (21) of the eye (17) is recorded using a frequency domain method.

3. The method according to claim 1 or 2,
**characterised in that**
by means of the second analysis system (28) for obtaining an image record, a depth profile of a cornea (21) of the eye (17) is recorded.

4. The method according to any one of the preceding claims,
**characterised in that**
the first and the second image data set are gathered at the same time.

5. The method according to any one of the preceding claims,
**characterised in that**
before supplementing the first image data set, the second image data set is corrected according to the first image data set.

6. The method according to claim 5,
**characterised in that**
the second image data set is corrected according to optical boundary surfaces which are established from the first image data set.

7. The method according to claim 5 or 6,
**characterised in that**
by means of the first analysis system (11), indices of refraction and/or a topography of a cornea (21) are established, which are taken into account in the correction.

8. The method according to any one of the preceding claims,
**characterised in that**
the first image data set is superimposed with the second image data set.

9. The method according to any one of the preceding claims,
**characterised in that**
a plurality of first and second image data sets is obtained in a sequential order, wherein the joint recording plane (18) of the first and of the second analysis system (11, 28) is pivoted about a visual axis (16) of the eye (17).

10. The method according to any one of the preceding claims,
**characterised in that**
a relative position of at least one optical boundary surface of the eye (17), which surface is established using the first analysis system (11), is determined as a reference surface for the second analysis system (28).

11. The method according to any one of the preceding claims,
**characterised in that**
the first analysis system (11) and the second analysis system (28) respectively emit light of different wavelength ranges.

12. An ophthalmological analysis system (10) for examining an eye in the region of a front eye section (20) of an eye (17), comprising a first analysis system (11) for obtaining first sectional images (22) of the eye, wherein the first analysis system is formed from a projection device (12) and from a monitoring device (13), which are arranged relative to each other according to the Scheimpflug rule, and comprising a second analysis system (28) for obtaining second sectional images (35) of the eye, wherein the second analysis system is formed from an optical coherence interferometer, wherein the ophthalmological analysis system comprises a processing device, wherein, from at least one coinciding recording plane (18) of the eye, a first image data set can be obtained using the first analysis system and a second image data set can be obtained using the second analysis system, wherein the processing device is constructed such that said device is able to process the first and the second image data set, wherein, by means of the processing device, the first image data set can, at least partially, be supplemented with data of the second image data set, wherein the second analysis system (28) comprises a beam guidance device (30), wherein the beam guidance device (30) has a movable tilting mirror (31) which is arranged in a measurement axis of the analysis system (10), and by means of which a measurement beam (33) of the optical coherence interferometer can be projected onto the cornea (21), wherein the measurement beam can be diverted within the recording plane (18) such that the eye (17) can be scanned by the measurement beam along the recording plane,
**characterised in that**
the beam guidance device (30) has a concave mirror (32), by means of which the measurement beam (33) can be deflected onto a cornea (21), wherein the concave mirror is constructed such that an optical path length of the measurement beam up to the cornea remains unchanged when scanning different points of the cornea.

13. The analysis system according to claim 12,
**characterised in that**
the beam guidance device (30) is constructed such that the measurement beam (33) can be projected onto a surface point (38) of a cornea (21), wherein the measurement beam can be projected onto the cornea at an angle which deviates from 90° relative to a tangent plane (37) of the surface point.

14. The analysis system according to claim 12 or 13,
**characterised in that**
beam paths (15, 29) of the first and of the second analysis system (11, 28) jointly run on the measurement axis, and wherein the tilting mirror (31) is a dichroic mirror.

15. The analysis system according to any one of claims 12 to 14,
**characterised in that**
a measurement range of the second analysis system (28) is adapted to a curvature of a cornea (21).

16. The analysis system according to any one of claims 12 to 15,
**characterised in that**
the joint recording plane (18) of the first and of the second analysis system (11, 28) can be pivoted about a visual axis (16) of the eye (17) by means of a rotation device, wherein the optical coherence interferometer has an optical fibre which is mechanically decoupled from the rotation device.

## Revendications

1. Procédé destiné à examiner un œil dans la région d'une partie avant (20) d'un œil (17), ayant un système d'analyse ophtalmologique (10), comprenant un premier système d'analyse (11) pour obtenir des premières images en coupe (22) de l'œil, ledit premier système d'analyse étant formé d'une unité de projection (12) et d'une unité d'observation (13) qui sont disposées l'une par rapport à l'autre selon la loi de Scheimpflug, et comprenant un deuxième système d'analyse (28) pour obtenir des deuxièmes images en coupe (35) de l'œil, ledit deuxième système d'analyse étant formé d'un interféromètre à cohérence optique, ledit système d'analyse ophtalmologique comprenant une unité de traitement, un premier ensemble de données d'images et un deuxième ensemble de données d'images étant obtenus respectivement à l'aide du premier système d'analyse et à l'aide du deuxième système d'analyse sur la base d'au moins un plan d'enregistrement (18) coïncidant de l'œil, ladite unité de traitement traitant le premier et le deuxième ensemble de données d'images, le premier ensemble de données d'images au moins en partie étant supplémenté par des données du deuxième ensemble de données d'images moyennant l'unité de traitement, ledit deuxième système d'analyse (28) comprenant une unité de guidage de faisceaux (30), ladite unité de guidage de faisceaux (30) présentant un miroir pivotant (31) mobile qui est disposé dans un axe de mesure du système d'analyse (10), et moyennant lequel un faisceau de mesure (33) de l'interféromètre à cohérence optique est projeté sur la cornée (21), ledit faisceau de mesure étant fait dévier dans le plan d'enregistrement (18) de telle façon que l'œil (17) est exploré le long du plan d'enregistrement par le faisceau de mesure,
**caractérisé en ce que**
l'unité de guidage de faisceaux (30) présente un miroir concave (32) moyennant lequel le faisceau de mesure (33) est dévié sur une cornée (21), ledit miroir concave étant réalisé de telle façon qu'une longueur du chemin optique du faisceau de mesure demeure inchangée jusqu'à la cornée pendant l'exploration de différents points de la cornée.

2. Procédé selon la revendication 1,
**caractérisé en ce**
**qu'**une image d'une cornée (21) de l'œil (17) est acquise moyennant le deuxième système d'analyse (28) à l'aide d'un procédé de domaine fréquentiel.

3. Procédé selon la revendication 1 ou 2,
**caractérisé en ce**
**qu'**une entretoise de profondeur d'une cornée (21) de l'œil (17) est enregistrée moyennant le deuxième système d'analyse (28) pour obtenir un enregistrement d'image.

4. Procédé selon l'une quelconque des revendications précédentes,
**caractérisé en ce que**
le premier et le deuxième ensemble de données d'images sont saisies en même temps.

5. Procédé selon l'une quelconque des revendications précédentes,
**caractérisé en ce que**
le deuxième ensemble de données d'images est corrigé selon le premier ensemble de données d'images avant la supplémentation du premier ensemble de données d'images.

6. Procédé selon la revendication 5,
**caractérisé en ce que**
le deuxième ensemble de données d'images est corrigé selon des surfaces de délimitation optiques qui sont déterminées à partir du premier ensemble de données d'images.

7. Procédé selon la revendication 5 ou 6,
**caractérisé en ce que**
des indices de réfraction et/ou une topographie d'une cornée (21) sont/est déterminé(s) moyennant le premier système d'analyse (11), lesdits indices de réfraction et ladite topographie étant pris en compte lors de la correction.

8. Procédé selon l'une quelconque des revendications précédentes,
**caractérisé en ce que**
le premier ensemble de données d'images est superposé avec le deuxième ensemble de données d'images.

9. Procédé selon l'une quelconque des revendications précédentes,
**caractérisé en ce**
**qu'**une pluralité de premiers et de deuxièmes ensembles de données d'images est obtenue dans une succession séquentielle, le plan d'enregistrement (18) commun du premier et du deuxième système d'analyse (11, 28) étant pivoté autour d'un axe visuel (16) de l'œil (17).

10. Procédé selon l'une quelconque des revendications précédentes,
**caractérisé en ce**
**qu'**une position relative d'au moins une des surfaces de délimitation optiques de l'œil (17) qui sont déterminées à l'aide du premier système d'analyse (11) est déterminée comme surface de référence pour le deuxième système d'analyse (28).

11. Procédé selon l'une quelconque des revendications précédentes,
**caractérisé en ce que**
le premier système d'analyse (11) et le deuxième système d'analyse (28) émettent de la lumière ayant des zones de longueur d'onde différentes.

12. Système d'analyse ophtalmologique (10) destiné à examiner un œil dans la région d'une partie avant (20) d'un œil (17), comprenant un premier système d'analyse (11) pour obtenir des premières images en coupe (22) de l'œil, ledit premier système d'analyse étant formé d'une unité de projection (12) et d'une unité d'observation (13) qui sont disposées l'une par rapport à l'autre selon la loi de Scheimpflug, et comprenant un deuxième système d'analyse (28) pour obtenir des deuxièmes images en coupe (35) de l'œil, ledit deuxième système d'analyse étant formé d'un interféromètre à cohérence optique, ledit système d'analyse ophtalmologique comprenant une unité de traitement, un premier ensemble de données d'images et un deuxième ensemble de données d'images pouvant être obtenus respectivement à l'aide du premier système d'analyse et à l'aide du deuxième système d'analyse sur la base d'au moins un plan d'enregistrement (18) coïncidant de l'œil, ladite unité de traitement étant réalisée de façon à pouvoir traiter le premier et le deuxième ensemble de données d'images, le premier ensemble de données d'images au moins en partie pouvant être supplémenté par des données du deuxième ensemble de données d'images moyennant l'unité de traitement, ledit deuxième système d'analyse (28) comprenant une unité de guidage de faisceaux (30), ladite unité de guidage de faisceaux (30) présentant un miroir pivotant (31) mobile qui est disposé dans un axe de mesure du système d'analyse (10), et moyennant lequel un faisceau de mesure (33) de l'interféromètre à cohérence optique peut être projeté sur la cornée (21), ledit faisceau de mesure pouvant être fait dévier dans le plan d'enregistrement (18) de telle façon que l'œil (17) peut être exploré le long du plan d'enregistrement par le faisceau de mesure,
**caractérisé en ce que**
l'unité de guidage de faisceaux (30) présente un miroir concave (32) moyennant lequel le faisceau de mesure (33) peut être dévié sur une cornée (21), ledit miroir concave étant réalisé de telle façon qu'une longueur du chemin optique du faisceau de mesure demeure inchangée jusqu'à la cornée pendant l'exploration de différents points de la cornée.

13. Système d'analyse selon la revendication 12,
**caractérisé en ce que**
l'unité de guidage de faisceaux (30) est réalisée de telle façon que le faisceau de mesure (33) peut être projeté sur un point (38) de la surface d'une cornée (21), ledit faisceau de mesure pouvant être projeté sur la cornée sous un angle différant de 90° par rapport à un plan tangent (37) du point de la surface.

14. Système d'analyse selon la revendication 12 ou 13,
**caractérisé en ce que**
des trajets des faisceaux (15, 29) du premier et du deuxième système d'analyse (11, 28) passent conjointement sur l'axe de mesure, et le miroir pivotant (31) étant un miroir dichroïque.

15. Système d'analyse selon l'une quelconque des revendications 12 à 14,
**caractérisé en ce**
**qu'**une plage de mesure du deuxième système d'analyse (28) est adaptée à une courbure d'une cornée (21).

16. Système d'analyse selon l'une quelconque des revendications 12 à 15,
**caractérisé en ce que**
le plan d'enregistrement (18) commun du premier et du deuxième système d'analyse (11, 28) peut être pivoté autour d'un axe visuel (16) de l'œil (17) moyennant une unité de rotation, l'interféromètre à cohérence optique présentant une fibre optique qui est découplée mécaniquement de l'unité de rotation.
